# FASCICULE DE BREVET EUROPEEN

(11) **EP 1 827 365 B1**
(45) Date de publication et mention de la délivrance du brevet: **20.09.2017**
(21) Numéro de dépôt: 05850571.0
(22) Date de dépôt: 21.12.2005
(51) Int. Cl.: A61K 8/27, A61K 8/44, A61K 8/60, A61K 8/64, A61K 8/67, A61K 8/23, A61Q 5/00, A61Q 7/00, A61K 8/19

(54) **UTILISATION D'UNE BASE NUTRITIVE COMPLEXE DANS LE DOMAINE COSMETIQUE, EN PARTICULIER CAPILLAIRE**
VERWENDUNG EINER KOMPLEXEN NÄHRSTOFFBASE IN KOSMETIKA; INSBESONDERE FÜR HAARE
USE OF A COMPLEX NUTRITIONAL BASE IN COSMETICS, IN PARTICULAR FOR THE HAIR

(30) Priorité: 21.12.2004 FR 0413658
(43) Date de publication de la demande: 05.09.2007
(73) Titulaire: Thorel, Jean-Noël, 75014 Paris (FR)
(72) Inventeur: GATTO, Hugues, F-06570 Saint-Paul (FR)
(74) Mandataire: Cabinet Laurent & Charras
(86) Numéro de dépôt international: PCT/FR2005/003228
(87) Numéro de publication internationale: WO 2006/067335

(56) Documents cités:
- EP-A- 0 383 467
- EP-A- 0 572 167
- WO-A-96/21421
- FR-A- 2 535 201
- FR-A- 2 694 692
- FR-A- 2 864 445
- US-A- 5 597 575

## Description

La présente invention concerne une base nutritive complexe (en abrégé BNC) en milieu aqueux, autrement appelée base éco-nutritive, adaptée pour le traitement du cheveu et/ou du cuir chevelu chez l'homme ou la femme, et ses diverses utilisations dans le domaine cosmétique capillaire.

Par "base nutritive complexe", on entend toute composition ou formulation, en milieu aqueux, se distinguant, comme précisé ci-après, d'un milieu de culture cellulaire, en ce qu'elle exclut tout facteur de croissance cellulaire, et/ou tout extrait d'origine animale ou cellulaire, non tracés, et se rapprochant d'un milieu de culture cellulaire, en ce qu'elle permet à elle seule une culture viable in vitro de kératinocytes épidermiques humains, par exemple pendant au moins 72 heures, avec au moins une prolifération clonale au premier passage, sans assise nourricière vivante, telle que fibroblastes.

De telles BNC ont été décrites dans le document WO/96/21421, qui décrit leur utilisation, seule ou en combinaison avec d'autres composants, en tant que produit actif ou comme excipient.

Une BNC telle que considérée selon la présente invention, ne comporte, ni dans sa composition originelle ni dans sa mise en oeuvre, de facteur de croissance d'origine cellulaire, par exemple d'EGF (Epidermal Growth Factor), et/ou d'extrait biologique d'origine animale ou cellulaire

Par nature, non seulement ces extraits ont une composition variable, voire indéterminée, avec parfois une origine biologique mal déterminée, et donc non traçable ou non tracée, mais également certains constituants sont indéterminés quant à leur structure chimique, notamment biochimique, exacte.

Une BNC telle que considérée selon la présente invention, ne comporte aucun extrait biologique tel que sérum de veau foetal (SVF), ou aucun extrait de tige pituitaire de boeuf, non traçable ou non tracée.

Par "tracer" ou "traçable", on entend la caractéristique selon laquelle l'origine puis le traitement d'une matière biologique peuvent être établis et contrôlés.

Une BNC selon la présente invention ne comporte par exemple aucun extrait biologique cellulaire, végétal ou animal, traçable ou non, tracé ou non.

Une BNC considérée selon la présente invention, ne comporte pas de principe actif médicamenteux, tel qu'un antibiotique.

De telles BNC sont constituées, outre le milieu aqueux, par une fraction d'acides aminés, dont certains essentiels, inférieure à 0,5%, préférentiellement à 0,35% en poids, par une fraction de vitamines hydrosolubles inférieure à 0,2%, et préférentiellement à 0,015% en poids, et par une fraction inorganique, dont oligo-éléments et sels métalliques, inférieure à 5%, et préférentiellement à 2% en poids, le solde de la composition étant représenté par de l'eau.

Préférentiellement, une BNC selon l'invention est entièrement formulé en phase aqueuse, à partir d'entités chimiques, biochimiques et biologiques, dont la structure de chacune est identifiée, par exemple nomenclaturée ou répertoriée, en sorte que la composition chimique de la BNC est strictement définie.

Une BNC selon l'invention résulte d'une action de la main de l'homme, et ne saurait être assimilée de ce fait à tout extrait naturel et/ou biologique, obtenu par exemple par fractionnement d'un matériel biologique, animal ou végétal par exemple.

Conformément au document WO 96/21421, une telle base nutritive complexe a, par exemple, la composition suivante, selon Tableau 1 ci-après :

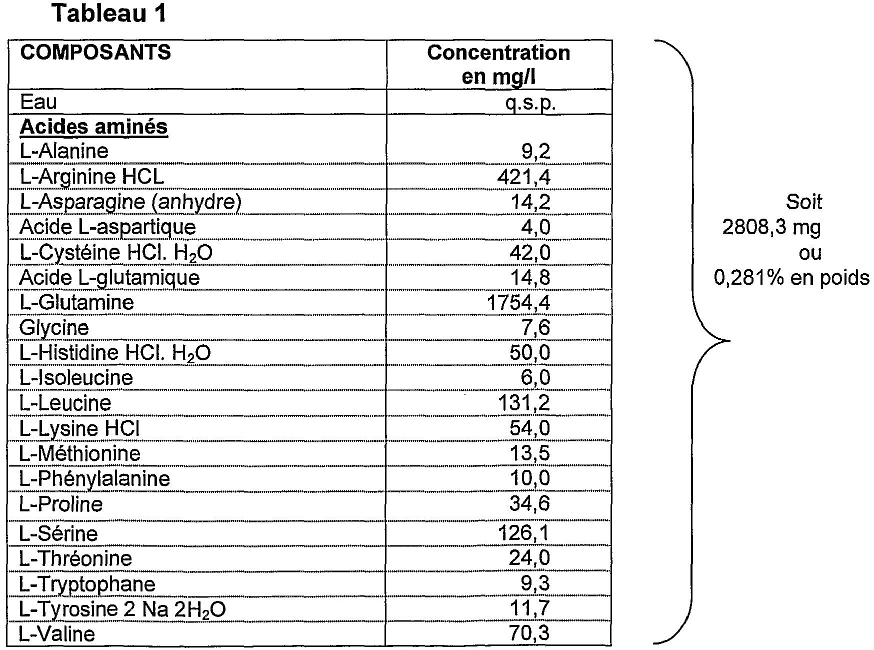

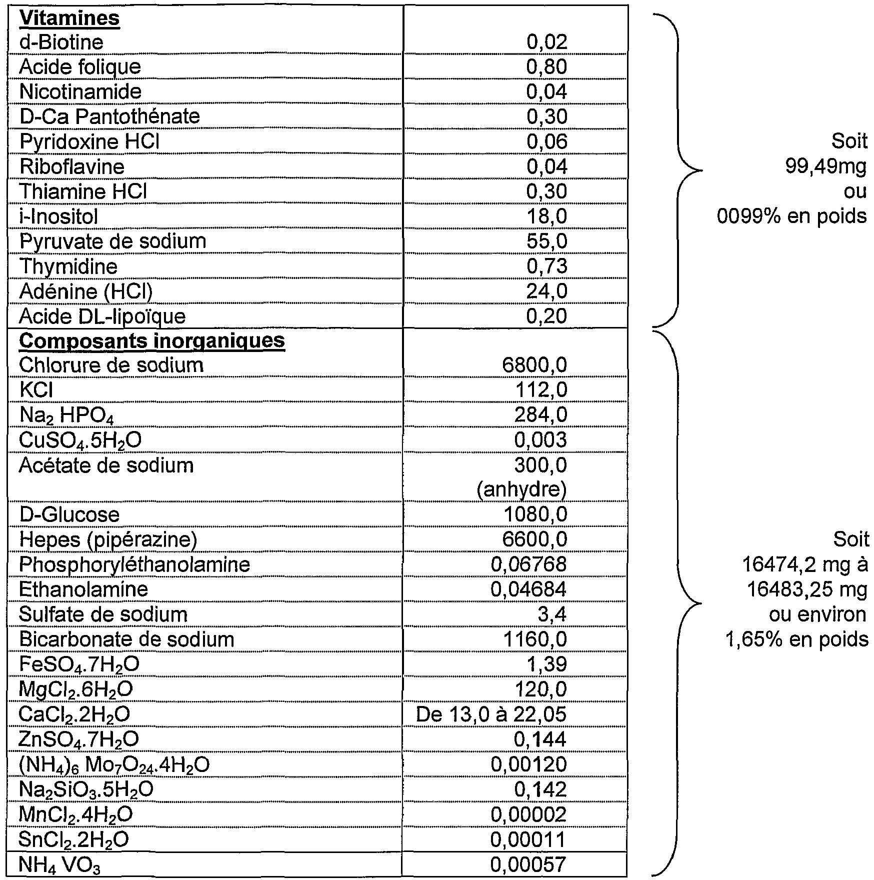

EP-A-0 383 467 décrit l'utilisation de sirop de molasses, issues de l'industrie sucrière, par application locale sur le cuir chevelu (ou autres zones pilaires), pour traiter les états pelliculaires et lutter contre la chute des cheveux.

Un sirop de molasse est un extrait naturel obtenu par fractionnement ou séparation à partir de la canne à sucre, dont la composition exacte demeure indéterminée ou variable selon l'origine, et ayant une forte teneur, représentant au moins 5% de la composition totale du sirop, en sucres, à savoir glucose, sucrose, fructose, et mono-oligosaccharides.

FR-A-2 535 201 décrit une composition ou préparation cosmétique destinée en particulier à favoriser la nutrition des follicules pileux. La partie active de cette composition consiste essentiellement en un milieu pour la culture in vitro de cellules épithéliales humaines isolées, complémenté avec du sérum foetal bovin.

Comme indiqué précédemment, un milieu de culture cellulaire, au surplus complémenté avec un sérum foetal bovin, n'est pas une BNC telle que envisagée par la présente invention.

Selon FR-A-2 535 201, pour favoriser le passage des substances nutritives du milieu de culture au travers de la peau, la composition cosmétique comprend un véhicule ou support dermophile, comprenant par exemple de l'alcool éthylique, des polyalcools ou polyglycols, et des émulsifiants, ainsi que des agents rubéfiants jouant le rôle de vasodilatateurs locaux.

Un tel véhicule ou support, non "écologique", apparaît incompatible avec une BNC telle que envisagée par la présente invention, et constituée pour l'essentiel par de l'eau, car il ne respecte pas les équilibres biologiques des parties superficielles du corps humain.

US-C-5 597 575 décrit une composition médicamenteuse topique, pour stimuler la repousse des cheveux, comportant à titre de principe actif une ou plusieurs vitamines liposolubles, telle que la vitamine D3 (ou certains de ses dérivés métaboliques actifs), préférentiellement fixées sur des micro-particules minérales non chargées.

A l'instar du Minoxidil, principe actif testé ci-après et qui est un vasodilatateur local, le mode d'action de cette composition résulte de l'irritation induite par la vitamine D3 au niveau du cuir chevelu, irritation provoquant une vasodilatation superficielle, laquelle stimule la croissance de la tige pilaire, car l'augmentation de la vascularisation de la papille dermique favorise l'apport de nutriments aux cellules de la matrice du follicule pileux.

Mais un tel effet vasodilatateur, généré par l'action irritante du principe actif, s'accompagne toujours d'une légère inflammation locale, inacceptable dans un traitement cosmétique.

La présente invention a pour objet de remédier aux inconvénients des solutions de l'art antérieur précédemment identifié.

Généralement, l'invention a pour objet d'adapter une BNC telle que précédemment définie, pour répondre spécifiquement aux besoins nutritionnels des kératinocytes des follicules pileux ou de la matrice de la tige du poil (zone de croissance de la tige pilaire située au-dessus de la papille dermique du follicule pileux).

Plus particulièrement, grâce à l'application locale d'une base ainsi adaptée, l'invention a pour objet un traitement cosmétique du cheveu et/ou du cuir chevelu chez l'homme ou la femme, agissant par simple mise en contact de la BNC avec le cuir chevelu ou le cheveu; et ce, en plus ou indépendamment de toute action mécanique, telle que massage du cuir chevelu, favorisant l'irrigation sanguine du bulbe pilaire ou la vascularisation du follicule pileux, et donc la croissance de la tige pilaire, et à l'exclusion de tout effet vasodilatateur, augmentant la vascularisation de la papille dermique, et favorisant à lui seul l'apport de nutriments aux cellules de la matrice du follicule pileux.

L'invention a également pour objet un traitement non thérapeutique du cheveu et/ou du cuir chevelu favorisant la pousse des cheveux, et stabilisant le phénomène de chute, à l'exclusion de toute activité pelliculaire.

Au premier chef, la présente invention a pour objet une base nutritive complexe en milieu aqueux se distinguant d'un milieu de culture cellulaire, en ce qu'elle exclut tout facteur de croissance cellulaire, et tout extrait biologique d'origine animale ou cellulaire, de composition indéterminée, non tracés, et se rapprochant d'un milieu de culture cellulaire, en ce qu'elle permet à elle seule une culture viable in vitro d'un inoculum de kératinocytes épidermiques humains, avec au moins une prolifération clonale de ces derniers au premier passage, sans assise nourricière vivante, ladite base étant constituée, outre le milieu aqueux, par une fraction d'acide aminé inférieure à 0,5%, et préférentiellement à 0,35% en poids, une fraction de vitamines hydrosolubles inférieure à 0,2%, et préférentiellement à 0,015%, et une fraction inorganique, dont oligo-éléments et sels métalliques, inférieure à 5% en poids, et préférentiellement à 2% en poids, selon la revendication 1. Selon l'invention, ladite base comprend une concentration pondérale totale en acide(s) aminé(s) soufré(s) suffisante pour permettre, dans des conditions standard in vitro, une augmentation de la synthèse des kératines de la tige pilaire du cheveu chez l'homme ou la femme.

Une telle BNC comporte en outre, au moins l'une des caractéristiques suivantes, lesquelles peuvent être considérées seules ou en combinaison :
- sa concentration pondérale totale en acide(s) aminé(s) soufré(s) est au plus égale à 104mg/l,
- sa concentration pondérale totale en acides aminés, dont acides aminés soufrés, est comprise entre 0,25 et 0,35%, et par exemple égale à environ 0,326%,
- la concentration pondérale de la fraction en vitamines est comprise entre 0,005 et 0,011%,
- sa concentration pondérale en composants organiques, dont oligo-éléments et sels métalliques, est comprise entre 1,25 et 1,35%, et par exemple égale à 1,347-1,348% en poids,
- sa concentration en glucose est comprise entre 0,1% et 0,6%, et par exemple comprise entre 0,45% et 0,6%,
- sa concentration en L-hydroxyproline est comprise entre 0,01% et 0,01%, et par exemple comprise entre 0,003% et 0,01%,
- sa concentration en acide ascorbique est comprise entre 0.00001%, et 0.001%, et par exemple comprise entre 0.0001% et 0,001%
- sa concentration en chacun des composés suivants, à savoir adénosine, guanine, deoxyribose et ribose, est comprise entre 0.000001% et 0.0001%, et par exemple comprise entre 0.00001 %.et 0,0001%,
- elle comprend une quantité cosmétiquement active d'au moins un inhibiteur de l'enzyme 5α-réductase de type I.
- l'inhibiteur de l'enzyme 5α-réductase de type I comprend un sel de zinc, par exemple du sulfate de zinc, et/ou de la vitamine B6.
- la base nutritive complexe comprend une quantité totale en calcium comprise entre 0 et 22,05 mg/l, et préférentiellement comprise entre 5 et 15 mg/l,
- la base nutritive complexe comprend des peptides extraits du lait, à une concentration comprise entre 0,01 et 1% en poids,
- le pH de la phase aqueuse est ajusté entre 7,4 et 7,5,
- l'osmolarité de la phase aqueuse est ajustée au plus entre 300 et 350 µOsm.

La présente invention a donc pour objet d'adapter une BNC, telle que précédemment considérée, à diverses applications cosmétiques, dont le traitement du cheveu et/ou du cuir chevelu chez l'homme ou la femme.

Au deuxième chef, l'invention concerne une composition cosmétique à usage local, en particulier pour le traitement chez l'homme du cheveu et/ou du cuir chevelu, comprenant une BNC telle que précédemment définie.

Au troisième chef, la présente invention concerne l'utilisation d'une BNC telle que précédemment définie, pour fabriquer ou obtenir une composition cosmétique adaptée à un traitement non thérapeutique du cheveu et/ou du cuir chevelu chez l'homme ou la femme.

Au quatrième chef, l'invention concerne un procédé de traitement non thérapeutique du cheveu et/ou du cuir chevelu, chez l'homme ou la femme, consistant de manière générale à appliquer localement et superficiellement sur le cuir chevelu une BNC telle que précédemment définie.

Un tel procédé permet de traiter l'alopécie androgénétique de l'adulte, chez l'homme ou chez la femme.

Un tel procédé permet de promouvoir la croissance des kératinocytes au niveau de la matrice de la tige du cheveu, et donc de favoriser la pousse des cheveux, et de stabiliser le phénomène de chute. Ce procédé est donc indiqué en cas de chute de cheveux modérée, et notamment en cas d'alopécie androgénétique.

Préférentiellement, la BNC est appliquée une à deux fois par jour sur le cuir chevelu de l'homme ou de la femme traitée.

La fonction inhibitrice de l'enzyme 5α-réductase de type I, de tout produit, composé, ou substance ou de toute quantité cosmétique active de ce dernier, peut être caractérisée ou mise en évidence selon l'enseignement de la publication :
- D. Stamatiadis et al, "Inhibition of 5α-réductase activity, in human skin by zinc and azelaic acid", British Journal of Dermatology (1988) 119, 627-632; cf en particulier pages 628-629.

Selon cette méthode de caractérisation :
- les matériaux utilisés sont la 1,2[³H]-testostérone, la [¹⁴C]- testostérone, la [¹⁴C]-dihydrotestostérone, et la [¹⁴C]-Δ4-androstenedione, et des [¹⁴C]- androstenediols, ainsi que du NADPH;
- la source in vitro de l'enzyme 5α-réductase est constituée par des prélèvements de peau sur des prépuces d'enfants de 2 à 3 mois;
- on incube des homogénats de cette peau en présence de concentrations croissantes de [³H]-testotérone et de NADPH, ainsi que le produit ou la substance candidate à l'inhibition ; après addition des [¹⁴C] stéroïdes, on sépare les métabolites, et on détermine les quantités de testotérone et de Δ4-androstenedione.

S'agissant des peptides naturels, ou d'origine naturelle, pouvant être mis en oeuvre selon la présente invention, on utilisera des fractions ou extraits obtenus à partir de lait d'origine animale, appelés ci-après "Milk Peptide Complex" ou MPC, par exemple l'extrait dit Whey Protein (Lactis Proteinum) ayant pour n° CAS 84082-51-9 et n° EINECS 281-998-7, fabriqué et vendu par la firme CLR-Chemisches Laboratorium en République Fédérale d'Allemagne.

Cet extrait présente à titre d'exemple les données analytiques suivantes :

| | |
|---|---|
| pH (0,5% en poudre MPC dans H₂O) | 5,0-7,0 |
| Perte à la dessication (2 h à 102°C) | 2,0-6,0% |
| Azote (Kjeldahl) | 1,4-1,9% |
| Protéines (Sigma Kit BCA-1) | 11,0-16,0% |
| Lactose (Boehringer Kit) | 65,0-71,0% |
| Acide (+) Lactique (Boehringer Kit) | 4,5-8,0% |
| Cendres (1000°C) | 6,0-9,0% |
| Graisse résiduelle | <0,5% |
| Elecrophorèse (SDS-PAGE) | correspond |
| Activité biologique-EC₅₀ (test de migration) | 50-500µg/ml |

S'agissant des acide(s) aminé(s) soufré(s) pouvant être utilisés selon la caractéristique de composition (f), leur concentration pondérale permettant d'augmenter la synthése des kératines de la tige pilaire, peut être déterminée par la mise en oeuvre du test décrit selon l'exemple 3.

S'agissant en particulier du traitement du cheveu et/ou du cuir chevelu, la mise en oeuvre de la présente invention apporte les avantages déterminants suivants :
- développer la croissance des kératinocytes au niveau de la matrice de la tige du cheveu, ce qui favorise la pousse ou la repousse des cheveux et stabilise le phénomène de chute, en particulier en cas d'alopécie androgénétique de l'adulte,
- il n'existe pratiquement pas d'effet secondaire ou indésirable, tel qu'une hypertrichose à distance.

Plus généralement, dans ses applications cosmétiques, une BNC selon la présente invention est d'utilisation particulièrement simple, non grasse et non collante. Elle est parfaitement tolérée localement, et dénuée d'effets secondaires et indésirables, tels que des irritations locales, sensations de brûlure et de prurit, un eczéma de contact, et dans certains cas modification de la pression artérielle et du pouls.

Une BNC selon la présente invention apparaît dénuée de cytotoxicité, quelle que soit sa concentration dans la composition dans laquelle elle appartient, par exemple avec un excipient.

La présente invention est maintenant décrite, à titre d'exemple, avec une composition pondérale selon Tableau 2.

En particulier, ci-après, dans son application capillaire, c'est-à-dire pour le traitement du cheveu et/ou du cuir chevelu, une BNC selon l'invention sera comparée à une spécialité pharmaceutique ayant la même indication, mais médicale, à savoir le Minoxidil (Dénomination Commune Internationale).

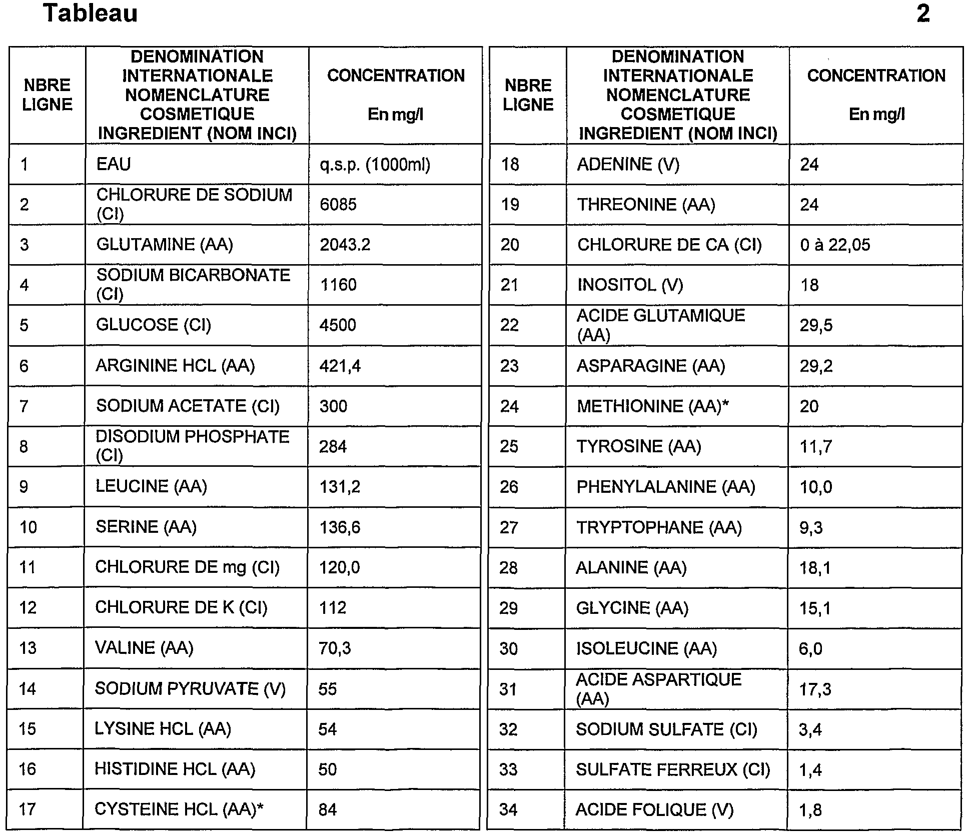

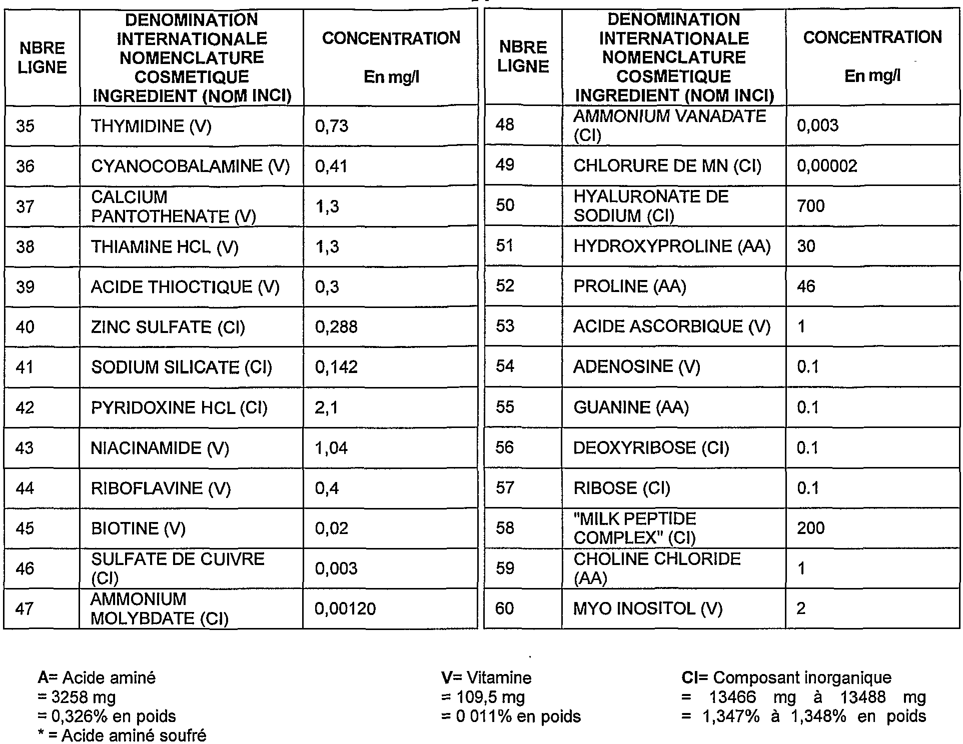

### Exemple 1

Il s'agit d'un essai concernant le Minoxidil, en tant que composé de référence, pour comparer ensuite l'efficacité d'une BNC selon l'invention, par rapport audit composé de référence.

L'objectif de l'essai est d'évaluer l'effet du Minoxidil (sous forme de sulfate hydrosoluble) sur la viabilité cellulaire de kératinocytes humains normaux.

La viabilité cellulaire des kératinocytes est appréciée par la technique de conversion du WST-1(*), qui consiste à évaluer l'activité du système mitochondrial succinate-tetrazolium reductase des cellules vivantes.

Le WST-1 (Boehringer/Roche) est réduit en un précipité coloré de formazan. La viabilité cellulaire est déterminée par lecture spectrophotométrique à 450 nm. L'intensité de la densité optique est proportionnelle au nombre de cellules vivantes.
(*) Sel de tetrazolium WST-1 : (4-(3-(4-lodophényl)-2-(4-nitrophényl)2H-5-tetrazolio)-1,3-benzene disulfonate).

Les résultats suivants sont obtenus :

Jusqu'à la concentration de 0,01%, on observe une absence d'effet cytotoxique du Minoxidil, après 24 et 48 h de contact. On peut même noter un effet de stimulation de la croissance cellulaire à la concentration de 0,001%.

Au-delà, on observe, à partir de 0,1%, un effet cytotoxique brutal, d'intensité similaire pour les 3 concentrations les plus élevées.

La méthode suivante est mise en oeuvre :

### Ensemencement

Les kératinocytes sont ensemencés dans des microplaques de 96 puits à raison de 20 000 cellules par puits dans 200 µl de culture de milieu standard KSFM (In Vitrogen). Les plaques sont incubées 24 h à 37°C en atmosphère humide contenant 6% de CO2.

### Gamme de concentrations testées

Les différentes concentrations à tester sont préparées à partir d'une solution -mère de Minoxidil (sulfate hydrosoluble) à 5% dans le PBS.

La gamme de concentrations testées s'étend de 0,0001 % à 2%.

### Traitement

Après élimination du milieu KSFM, les différentes dilutions de Minoxidil sont mises au contact des cellules. Les milieux ne sont pas renouvelés au cours de l'expérience.

Chaque point est réalisé en quadruplicate.

La cytoxicité (test au WST-1) est mesurée après 24 et 48 h de contact.

### Analyse

La densité optique est lue au lecteur de microplaque ELISA à 450 mm.

### Exemple 2

L'objectif de cet essai est d'évaluer la croissance de kératinocytes humains normaux, ensemencés à faible densité dans la BNC dont la composition est décrite par le Tableau 2.

L'étude est réalisée dans cette BNC, avec 0,2 mg/ml de complexe MPC (et sans complexe MPC), versus le milieu de culture standard des kératinocytes, le KSFM.

On procède de la manière suivante :

Les kératinocytes sont ensemencés à faible densité en plaque 96 puits dans le milieu standard KSFM, et poussent pendant 24 h après ensemencement dans ce milieu.

Au 2ème jour, les cellules sont placées dans les différents milieux étudiés :
- KSFM
- BNC selon tableau 2, sans complexe MPC
- BNC selon tableau 2, avec complexe MPC à 0,2 mg/ml

Chaque condition est réalisée en quadruplicate. Les milieux sont renouvelés tous les 3 jours au cours de l'expérimentation.

La densité cellulaire est évaluée 24h après l'ensemencement des cellules, avant la mise au contact des différentes conditions d'étude (= T0), puis la croissance des kératinocytes est évaluée aux 2^{ème}, 4^{ème}, 6^{ème} et 8^{ème} jours de culture par la méthode de conversion du WST-1 (lecture à 450 nm).

La croissance cellulaire est objectivée par la mesure de la viabilité cellulaire à différents temps de l'expérimentation :
- En présence de complexe MPC à la concentration de 0,2mg/ml, on observe une croissance cellulaire continue des kératinocytes avec la BNC selon le Tableau 2.
- Sans ajout de complexe MPC, on observe une absence de croissance cellulaire avec la BNC selon le Tableau 2 (en conditions hypocalciques).

En conclusion, dans les conditions expérimentales ainsi définies, on observe avec une BNC comprenant un complexe MPC à la concentration de 0,2mg/ml, une croissance cellulaire régulière des kératinocytes humains normaux sur 8 jours de culture, d'intensité supérieure à celle observée avec la même BNC, mais sans ledit complexe.

### Exemple n° 3

Il s'agit d'évaluer l'effet de la BNC selon Tableau 1 sur la prolifération et la différenciation de fragments de cuir chevelu maintenus en survie.

Le but de cet essai est de mettre en évidence l'efficacité de la base selon le Tableau 1 sur la survie ex vivo de fragments de cuir chevelu humain. L'évaluation de la prolifération à l'aide de l'anticorps Ki67 a été réalisée au niveau de la gaine épithéliale externe entourant le cheveu. L'évaluation de la différenciation des kératinocytes à l'aide d'un anticorps anti-cytokératines totales a été effectuée au niveau de la tige pilaire du cheveu et plus particulièrement au niveau de sa corticale.

Les matériel et méthodes suivants sont mis en oeuvre :

### 1) Maintien en survie de cuir chevelu en présence de la BNC selon Tableau 1

Des fragments de cuir chevelu de 5 donneurs différents (patients atteints d'alopécie androgénique, prélevés à la jonction entre la zone où persistent les cheveux et la zone alopécique) ont été déposés dans des inserts eux-mêmes positionnés sur des puits de culture.

La BNC étant sans conservateur, des antibiotiques ont été ajoutés (fungizone, gentamycine). L'efficacité de la BNC sur la survie et la différenciation des fragments de peau a été comparée avec celle obtenue en présence d'un tampon phosphate type PBS. La BNC selon Tableau 1 versus PBS a été ajoutée quotidiennement dans le fond des puits, un passage s'effectuant par diffusion lente entre les deux compartiments par l'intermédiaire d'une membrane poreuse (12µm)

Les fragments du cuir chevelu ont été maintenus en survie dans une étuve à 37°C et en atmosphère air/CO₂ 5% pendant 48 heures.

### Analyses

Les fragments du cuir chevelu sont fixés dans le liquide de Bouin et inclus en paraffine.

L'évaluation immunohistochimique de la prolifération a été effectuée au niveau de la gaine épithéliale externe entourant le cheveu. Cette gaine épithéliale externe est considérée histologiquement comme le prolongement de l'épiderme de surface. L'évaluation de la différenciation a été réalisée au niveau de la corticale de la tige pilaire. En effet, la tige pilaire est constituée dans sa partie centrale de la médullaire (colonne centrale faite de cellules sans noyau), de la corticale faite de cellules kératinisées contenant des pigments mélaniques et de la cuticule.

### a) Analyse immunohistochimique de l'activité mitotique de la gaine épithéliale externe des follicules pileux

La prolifération épithéliale a été analysée par immunohistochimie à l'aide d'un anticorps anti-Ki67 (marqueur des cellules en phases M, S, G1 et G2 du cycle cellulaire). L'immunodétection a été réalisée à l'aide d'une technique d'immunoperoxydase indirecte en 3 couches, amplifiée (kit DAKO) et révélée en DAB.

Le nombre de cellules marquées est évalué au niveau de la gaine épithéliale externe des sections de bulbe pilaire présentes au niveau du cuir chevelu (8 à 10 par coupe). Le pourcentage de cellules en cours de prolifération a ainsi été calculé.

### b) Analyse immunohistochimique de la différenciation épithéliale de la corticale de la tige pilaire

La différenciation épithéliale est mise en évidence avec un anticorps anti-cytokératines totales (Novocastra).

L'immunodétection est réalisée à l'aide d'une technique d'immunoperoxydase indirecte en 3 couches (kit ABC Peroxydase, Vector Laboratories) et révélée en DAB (Diaminobenzidine).

L'intensité du marquage immunohistochimique a été évaluée à l'aide des scores semi-quantitatifs suivants au niveau de tous les follicules pileux de la coupe de cuir chevelu examiné:
- négatif: score O -
- léger: score 1
- modéré: score 2
- important: score 3
- très important: score 4

### 3) Statistiques

L'analyse statistique a été effectuée par le test de Student dit de l'écart réduit ou test des échantillons appariés. Le seuil de signification est fixé à 5%.

Les résultats suivants sont obtenus :

### a) Analyse immunohistochimique de l'activité mitotique au niveau de la gaine épithéliale externe des follicules pileux

L'analyse de l'activité mitotique est exposée dans le tableau I ci-après.

Le traitement par la BNC selon le Tableau 1 a permis d'augmenter significativement le renouvellement des cellules épithéliales (p < 0,05). En effet, après traitement, 7,6% des cellules de la gaine épithéliale externe sont marquées par l'anticorps anti-Ki67, contre 2,6% pour les cuirs chevelus traités par le PBS. On a également réalisé une analyse de l'index mitotique au niveau de l'épiderme de surface. Les résultats sont également en faveur de la BNC selon le Tableau 1, avec un index de prolifération de 6,4% versus 1,1% (p < 0,05).

### b) Analyse immunohistochimique de la différenciation épithéliale au niveau de la corticale de la tige pilaire

L'analyse de la différentiation épithéliale est exposée dans le tableau II ci-après.

La différenciation épithéliale tend à être de meilleure qualité au niveau des cuirs chevelus traités par la BNC selon Tableau 1, en comparaison 16 avec ceux traités par PBS : le score global obtenu est de 3,3 contre 2,4. On n'a pas obtenu de différence significative, l'analyse au cas par cas permettant malgré tout d'observer une différenciation nettement meilleure dans 3 cas traités par la BNC selon Tableau 1 sur 5 et une diffétenciation non modifiée pour 1 cas.

**tableau I :**

| Prolifération cellulaire (immunohistochimie à l'aide de l'anticorps anti-Ki67): % de cellules marquées de l'épiderme de surface et de la gaine épithéliale externe des follicules pileux | | |
|---|---|---|
| | **épiderme** | **Gaine épithéliale externe** |
| Cuir chevelu + BNC selon Tableau 1 | 6,4 ±2,7* | 7,6 ± 1,9* |
| Cuir chevelu + PBS | 1,1 ± 0,9 | 2,6 ± 1,9 |

| | | |
|---|---|---|
| *: comparaison entre la BNC selon Tableau 1 et le contrôle PBS: différence statistiquement significative (test de Student apparié unilatéral, p < 0,05) | | |

**tableau II :**

| Différenciation épithéliale (immunohistochimie à l'aide d'un anticorps anti-cytokératines totales) de la corticale des tiges pilaires | |
|---|---|
| Cuir chevelu + BNC selon tableau 1 | 2,4 ± 1 |
| Cuir chevelu + PBS | 3,3 ± 0,9 |

En conclusion, le traitement par la BNC selon Tableau 1 permet d'obtenir une augmentation statistiquement significative de l'index mitotique au niveau de la gaine épithéliale externe des follicules pileux. La différenciation épithéliale de la corticale des tiges pilaires est améliorée, mais cependant sans caractère significatif.

### Exemple n° 4

Cet essai a pour objet de tester l'effet de la BNC selon Tableau 2 sur la stimulation du bulbe pilaire (prolifération cellulaire) a partir de fragments de cuir chevelu maintenus en survie.

Selon l'exemple n° 3, on a mis en évidence une augmentation statistiquement significative de l'index mitotique au niveau de la gaine épithéliale externe après maintien en survie de follicules pileux en présence d'une BNC selon Tableau 1.

Le but de la présente étude est de mettre en évidence l'efficacité d'une BNC selon l'invention et selon Tableau 2, sur la stimulation du bulbe pilaire. L'évaluation de la prolifération cellulaire, à l'aide de l'anticorps anti-Ki67, a été réalisée au niveau de la zone matricielle du bulbe (zone de croissance de la tige pilaire), mais également au niveau de la gaine épithéliale externe du follicule pileux.

Les matériels et méthodes suivants sont mis en oeuvre

### 1) Maintien en survie de cuir chevelu en présence d'une BNC selon Tableau 2

Des fragments de cuir chevelu de 6 donneurs différents (lift cervico-facial de sujet non alopécique) ont été déposés dans des inserts eux-mêmes positionnés sur des puits de culture.

L'efficacité de la BNC selon Tableau 2 sur la survie et la stimulation des bulbes pilaires a été comparée, d'une part, avec celle obtenue en présence d'un tampon phosphate type PBS, et d'autre part, en présence d'un composé de référence testé à 2 concentrations, le Minoxidil à 0,01 et 2% (dilution dans le tampon PBS), Minoxidil 0,01% étant une dose non cytotoxique sur cultures en monocouche de kératinocytes humains normaux, et 2% étant la concentration utilisée in vivo chez l'homme, pour la plupart des spécialités commercialisées dans le traitement de l'alopécie. Etant sans conservateur, des antibiotiques ont été ajoutés dans tous ces milieux testés (fungizone, gentamycine). Ils ont été ajoutés quotidiennement dans le fond des puits, un passage s'effectuant par diffusion lente entre les deux compartiments par l'intermédiaire d'une membrane poreuse (12 µm).

Les fragments de cuir chevelu sont maintenus en survie dans une étuve à 37°C et en atmosphère air/C0₂ 5% pendant 48 heures.

### 2) Analyse immunohistochimique de l'activité mitotique au niveau du bulbe pilaire et de la gaine épithéliale externe du follicule pileux

Les fragments de cuir chevelu sont fixés dans le liquide de Bouin et inclus en paraffine.

L'évaluation immunohistochimique de la prolifération a été effectuée au niveau du bulbe pilaire ainsi qu'au niveau de la gaine épithéliale externe entourant le cheveu. Cette gaine épithéliale externe est considérée histologiquement comme le prolongement de l'épiderme de surface.

La prolifération épithéliale a été analysée par immunohistochimie à l'aide d'un anticorps anti-Ki67 (marqueur des cellules en phases M, S, G1 et G2 du cycle cellulaire). L'immunodétection a été réalisée à l'aide d'une technique d'immunoperoxydase indirecte en 3 couches, amplifiée (kit DAKO) et révélée en AEC.

Le nombre de cellules marquées de la gaine épithéliale externe est comptabilisé entre l'origine des glandes sébacées et le début de la zone bulbaire. D'autre part, les cellules matricielles marquées dans les bulbes pilaires sont également dénombrées. Le pourcentage de cellules en cours de prolifération a ainsi été calculé par rapport aux cellules non marquées.

### 3) Statistiques

L'analyse statistique a été effectuée par le test de Student dit de l'écart réduit ou test des échamillons appariés. Le seuil de signification est fixé à 5%.

Les résultats suivants sont obtenus :

### a) Analyse immunohistochimique de l'activité mitotique au niveau des bulbes pilaires

L'analyse de l'activité mitotique est exposée dans le tableau I ci-après.

L'incubation de fragments de cuir chevelu en présence de la BNC selon Tableau 2 induit une augmentation significative de l'index mitotique au niveau des cellules matricielles du bulbe pilaire (15,2% de cellules marquées), en comparaison avec du tampon PBS (0,26% de cellules positives) ou du Minoxidil 0,01% (4,2% de cellules positives) (p < 0,05). Le taux de prolifération obtenu avec la BNC selon Tableau 2 est proche de celui observé avec du Minoxidil à 2% (11,60% de cellules positives).

### b) Analyse immunohistochimique de l'activité mitotique au niveau de la gaine épithéliale externe des follicules pileux

L'analyse de l'activité mitotique est exposée dans le tableau II ci-après.

L'incubation de fragments de cuir chevelu en présence de la BNC selon Tableau 2 induit une augmentation significative de l'index mitotique au niveau des kératinocytes de la gaine épithéliale externe des follicules pileux (22,3% de cellules marquées), en comparaison avec du tampon PBS (2,6% de cellules positives) (p < 0,05). Selon l'exemple n° 3, le traitement par une BNC selon Tableau 1 avait déjà permis d'augmenter significativement le renouvellement des cellules au niveau de la gaine épithéliale, mais avec une efficacité nettement inférieure à celle mesurée selon Tableau 2 (p < 0,05). En effet, après traitement, seuls 7,6% des cellules de la gaine épithéliale externe étaient marquées par l'anticorps anti-Ki67, contre 2,6% pour les cuirs chevelus traités par le PBS.

**tableau I :**

| **Prolifération cellulaire** (immunohistochimie à l'aide de l'anticorps anti-Ki67) : % de cellules matricielles marquées au niveau du bulbe du follicule pileux | |
|---|---|
| N=6 | % |
| Cuir chevelu + **BNC selon Tableau 2** | **15,2** ± 6,7*# |
| Cuir chevelu + **PBS** | **0,26** ± 0,6 |
| Cuir chevelu + **Minoxidil 0,01%** | **4,2** ± 2,45 |
| Cuir chevelu + **Minoxidil 2%** | **11,6** ± 8,7 |

| | |
|---|---|
| * comparaison entre BNC selon Tableau 2 et PBS: différence statistiquement significative (test de Student apparié unilatéral, p < 0,05) # comparaison entre BNC selon Tableau 2 et Minoxidil 0,01% : différence statistiquement significative (test de Student apparié unilatéral, p < 0,05) | |

**tableau II**

| **Prolifération cellulaire** (immunohistochimie à l'aide d'un anticorps anti-Ki67) : % de cellules marquées au niveau de la gaine épithéliale externe des follicules pileux | |
|---|---|
| N=6 | % |
| Cuir chevelu + **BNC selon Tableau 2** | **22,3** ± 4,4*# |
| Cuir chevelu + **PBS** | **2,6** ± 2,7 |
| Cuir chevelu + **Minoxidil 0,01%** | **10,2** ± 5 |
| Cuir chevelu + **Minoxidil 2%** | **17,6** ± 7# |

| | |
|---|---|
| * comparaison entre BNC selon Tableau 2 et PBS: différence statistiquement significative (test de Student apparié unilatéral, p < 0,05) # comparaison entre BNC selon Tableau 2 et Minoxidil 0,01% : différence statistiquement significative (test de Studient apparié unilatéral, p < 0,05) # comparaison entre Minoxidil 0,01% et Minoxidil 2% : différence statistiquement significative (test de Studient apparié unilatéral, p < 0,05). | |

| N = 5, pour rappel | % |
|---|---|
| Cuir chevelu + **BNC selon Tableau 1** | **7,6** ±1,9* |
| Cuir chevelu + **PBS** | **2,6** ± 1,9 |

Dans cette étude, on met en évidence une augmentation significative de la prolifération des kératinocytes au niveau de la gaine épithéliale externe des follicules pileux avec la BNC selon Tableau 2, en comparaison avec le Minoxidil 0,01% (10,2% de cellules positives) (p < 0,05). Ce taux de prolifération cellulaire (22,3%) est proche de celui obtenu avec le Minoxidil 2% (17,6% de cellules positives).

**En conclusion, avec un modèle de fragment de cuir chevelu maintenu en survie, le traitement par une BNC selon Tableau 2 permet d'obtenir une augmentation statistiquement significative de l'index mitotique des cellules matricielles du bulbe pilaire et des kératinocytes de la gaine épithéliale externe des follicules pileux. Le résultat est nettement supérieur à celui obtenu avec une BNC selon Tableau 1.**

## Revendications

1. Base nutritive complexe en milieu aqueux destinée à être utilisée dans un traitement pour favoriser la pousse des cheveux et stabiliser le phénomène de chute, se distinguant d'un milieu de culture cellulaire, en ce qu'elle exclut tout facteur de croissance cellulaire, et tout extrait biologique d'origine animale ou cellulaire, de composition indéterminée, non tracés, se rapprochant d'un milieu de culture cellulaire, et constituée, outre le milieu aqueux, par une fraction d'acides aminés inférieure à 0,5%, et préférentiellement à 0,35% en poids, par une fraction de vitamines hydrosolubles inférieure à 0,2%, et préférentiellement à 0,015%, et par une fraction inorganique, dont oligo-éléments et sels métalliques, inférieure à 5% en poids, et préférentiellement à 2% en poids, et en ce qu'elle comprend en outre les caractéristiques suivantes :
- une concentration pondérale totale en acide(s) aminé(s) soufré(s) au plus égale à 104 mg/l
- des peptides extraits de lait à une concentration comprise entre 0,01 et 1% en poids.
- la concentration pondérale totale en acides aminés, dont acides aminés soufrés, est comprise entre 0,25 et 0,35%,
- la concentration pondérale de la fraction en vitamines est comprise entre 0,005 et 0,015%,
- la concentration pondérale en composants inorganiques, dont oligo-éléments et sels métalliques, est comprise entre 1,25 et 1,35%,
- la concentration en glucose est comprise entre 0,1% et 0,6%,
- la concentration en L hydroxyproline est comprise entre 0,003% et 0,01 %,
- la concentration en acide ascorbique est comprise entre 0,00001% et 0,001%,
- la concentration en chacun des composés suivants, à savoir adénosine, guanine, deoxyribose et ribose, est comprise entre 0,000001% et 0,0001%,
- elle comprend au moins un inhibiteur de l'enzyme 5α-réductase de type I,
- elle comprend une quantité totale en calcium comprise entre 0 et 22,05 mg/l.

2. Base selon la revendication 1, **caractérisée en ce que** la concentration pondérale totale en acides aminés, dont acides aminés soufrés est - égale à environ 0,326%.

3. Base selon la revendication 1, **caractérisée en ce que** la concentration pondérale de la fraction en vitamines est égale à environ 0,011%.

4. Base selon la revendication 1, **caractérisée en ce que** la concentration pondérale en composants inorganiques, dont oligo-éléments et sels métalliques, est égale à 1,347-1,348% en poids.

5. Base selon la revendication 1, **caractérisée en ce que** sa concentration en glucose est comprise entre 0,45% et 0,6%.

6. Base selon la revendication 1, **caractérisée en ce que** sa concentration en acide ascorbique est comprise entre 0,0001 % et 0,001 %.

7. Base selon la revendication 1, **caractérisée en ce que** sa concentration en chacun des composés suivants, à savoir adénosine, guanine, deoxyribose et ribose, est comprise entre 0,00001% et 0,0001%.

8. Base selon la revendication 1, **caractérisée en ce que** l'inhibiteur de l'enzyme 5α-réductase de type I est unsel de zinc, par exemple du sulfate de zinc, et/ou de la vitamine B6.

9. Base selon la revendication 1, **caractérisée en ce qu'**elle comprend une quantité totale en calcium comprise entre 5 et 15 mg/l.

10. Base selon la revendication 1, **caractérisée en ce que** le pH de la phase aqueuse est ajusté entre 7,4 et 7,5.

11. Base selon la revendication 1, **caractérisée en ce que** l'osmolarité de la phase aqueuse est ajustée au plus entre 300 et 350 µOsm.

12. Base nutritive complexe selon l'une des revendications 1 à 11 destinée à être utilisée en tant que médicament pour le traitement de l'alopécie androgénétique de l'adulte, chez l'homme ou chez la femme.

## Patentansprüche

1. Komplexe Nährstoffbasis in wässrigem Medium, die zur Verwendung bei einer Behandlung zur Begünstigung des Wachstums der Haare und Stabilisierung der Erscheinung von Haarausfall ausgelegt ist, die sich von einem Zellkulturmedium dadurch unterscheidet, dass sie jeden Zellwachstumsfaktor und jeden biologischen Extrakt tierischer oder zellulärer Herkunft ausschließt, unbestimmter, nicht vorgeschriebener Zusammensetzung, näherungsweise ein Zellkulturmedium, und bestehend, neben dem wässrigen Medium, aus einer Fraktion von Aminosäuren von weniger als 0,5 %, und vorzugsweise weniger als 0,35 Gew.-%, aus einer Fraktion von wasserlöslichen Vitaminen von weniger als 0,2 %, und vorzugsweise weniger als 0,015%, und aus einer anorganischen Fraktion, deren Oligo-Elemente und Metallsalze weniger als 5 Gew.-% und vorzugsweise weniger als 2 Gew.-% betragen, und **dadurch, dass** sie weiterhin die folgenden Merkmale aufweist:
- eine Gewichts-Gesamtkonzentration an schwefelhaltiger(n) Aminosäure(n) von höchstens gleich 104 mg/L
- aus Milch extrahierte Peptide in einer Konzentration von 0,01 bis 1 Gew.-%.
- die Gewichts-Gesamtkonzentration an Aminosäuren, davon schwefelhaltige Aminosäuren, beträgt 0,25 bis 0,35 %,
- die Gewichtskonzentration der Fraktion an Vitaminen beträgt 0,005 bis 0,015%,
- die Gewichtskonzentration an anorganischen Bestandteilen, davon Oligo-Elemente und Metallsalze, beträgt 1,25 bis 1,35 %,
- die Konzentration an Glucose beträgt 0,1 % bis 0,6 %,
- die Konzentration an L-Hydroxyprolin beträgt 0,003 % bis 0,01 %,
- die Konzentration an Ascorbinsäure beträgt 0,00001 % bis 0,001 %,
- die Konzentration von jedem der folgenden Bestandteile, und zwar Adenosin, Guanin, Deoxyribose und Ribose, beträgt 0,000001 % bis 0,0001 %,
- sie umfasst mindestens einen Inhibitor des Enzyms 5α-Reduktase vom Typ I,
- sie umfasst eine Gesamtmenge an Calcium von 0 bis 22,05 mg/L.

2. Basis nach Anspruch 1, **dadurch gekennzeichnet, dass** die Gewichts-Gesamtkonzentration an Aminosäuren, davon schwefelhaltige Aminosäuren - etwa gleich 0,326 % beträgt.

3. Basis nach Anspruch 1, **dadurch gekennzeichnet, dass** die Gewichtskonzentration der Fraktion an Vitaminen etwa gleich 0,011 % beträgt.

4. Basis nach Anspruch 1, **dadurch gekennzeichnet, dass** die Gewichtskonzentration an anorganischen Bestandteilen, davon Oligo-Elemente und Metallsalze, gleich 1,347-1,348 Gew.-% beträgt.

5. Basis nach Anspruch 1, **dadurch gekennzeichnet, dass** ihre Konzentration an Glucose 0,45 % bis 0,6 % beträgt.

6. Basis nach Anspruch 1, **dadurch gekennzeichnet, dass** ihre Konzentration an Ascorbinsäure 0,0001 % bis 0,001 % beträgt.

7. Basis nach Anspruch 1, **dadurch gekennzeichnet, dass** ihre Konzentration an jedem der folgenden Bestandteile, und zwar Adenosin, Guanin, Deoxyribose und Ribose, 0,00001 % bis 0,0001 % beträgt.

8. Basis nach Anspruch 1, **dadurch gekennzeichnet, dass** der Inhibitor des Enzyms 5α-Reduktase vom Typ I ein Zinksalz ist, beispielsweise Zinksulfat und /oder Vitamin B6.

9. Basis nach Anspruch 1, **dadurch gekennzeichnet, dass** sie eine Gesamtmenge an Calcium von 5 bis 15 mg/L umfasst.

10. Basis nach Anspruch 1, **dadurch gekennzeichnet, dass** der pH der wässrigen Phase zwischen 7,4 und 7,5 eingestellt ist.

11. Basis nach Anspruch 1, **dadurch gekennzeichnet, dass** die Osmolarität der wässrigen Phase zwischen höchstens 300 und 350 µOsm eingestellt ist.

12. Komplexe Nährstoffbasis nach einem der Ansprüche 1 bis 11, die zur Verwendung als Medikament zur Behandlung des androgenetischen Haarausfalls beim erwachsenen Mann oder bei der erwachsenen Frau bestimmt ist.

## Claims

1. A complex nutrient base in aqueous medium for use in a treatment to promote hair growth and stabilize the phenomenon of hair loss, being distinguished from a cell culture medium, that the complex nutrient base excludes any cell growth factor and any biological extract of animal or cellular origin having an untraced, an indeterminant composition, closer to a cell culture medium , which is constituted, further the aqueous medium, by an amino acid fraction less than 0.5%, and preferably less than 0.35% in weight, by a water-soluble vitamin fraction less than 0.2%, and preferably less than 0.015%, and by an inorganic fraction, including trace elements and metal salts, less than 5% in weight, and preferably less than 2% in weight, and further comprising following features:
- a total weight concentration of sulfur-bearing amino acids is not higher than 104 mg/l,
- peptides extracted from milk at a concentration between 0.01 and 1% in weight,
- the total weight concentration of amino acids, including sulfur-beraing amino acids is between 0.25 and 0.35%,
- the weight concentration of the vitamins fraction is between 0.005 and 0.015%,
- the weight concentration of inorganic components, including trace elements and metal salts , is between 1.25 and 1.35%,
- the glucose concentration is between 0.1% and 0.6%,
- the L-hydroxyproline concentration is between 0.003% and 0.01%,
- the ascorbic acid concentration is between 0.00001% and 0.001%,
- the concentration of each of following compounds, namely adenosine, guanine, deoxyribose and ribose, is between 0.000001% and 0.0001%,
- it comprises at least one type I 5α-reductase enzyme inhibitor,
- it comprises a total quantity of calcium between 0 and 22.05 mg/l.

2. The complex nutrient base according to claim 1, **characterized in that** the total weight concentration in amino acids, including sulfur-bearing acimo acids is equal to about 0.326%.

3. The complex nutrient base according to claim 1, **characterized in that** the weight concentration of vitamins fraction is equal to about 0.011 %.

4. The complex nutrient base according to claim 1, **characterized in that** the weight concentration of inorganic compounds, including trace elements and metal salts, is equal to 1.347-1.348% in weight.

5. The complex nutrient base according to claim 1, **characterized in that** the glucose concentration is between 0.45% and 0.6%.

6. The complex nutrient base according to claim 1, **characterized in that** the ascorbic acid concentration is between 0.0001% and 0.001%.

7. The complex nutrient base according to claim 1, **characterized in that** its concentration in each of following compounds, namely adenosine, guanine, deoxyribose and ribose, is between 0.00001% and 0.0001%.

8. The complex nutrient base according to claim 1, **characterized in that** the type I 5α-reductase enzyme inhibitor is a zinc salt, for example, zinc sulfate, and/or vitamin B6.

9. The complex nutrient base according to claim 1, **characterized in that** it comprises a total quantity of calcium between 5 and 15 mg/l.

10. The complex nutrient base according to claim 1, **characterized in that** the pH of aqueous phase is adjusted to between 7.4 and 7.5.

11. The complex nutrient base according to claim 1, **characterized in that** the osmolarity of aqueous phase is adjusted to not more than 300 and 350 µOsm.

12. The complex nutrient base according to one of claims 1 to 11 for its use as a medicament for treating adult androgenetic alopecia, of man or woman.
